# EUROPEAN PATENT APPLICATION

(11) **EP 4 802 986 A1**
(43) Date of publication of application: **09.09.2026**
(21) Application number: 26162041.3
(22) Date of filing: 03.03.2026
(51) Int. Cl.: A61B 1/00, A61B 1/12

(54) **A NOVEL ENDOSCOPIC ULTRASONIC CLEANING ADAPTER**

(30) Priority: 07.03.2025 US 202563768311 P
(71) Applicant: GA HEALTH COMPANY LIMITED, Shatin, N.T. (HK)
(72) Inventor: MCCABE, Ken, Hong Kong (CN); CHAN, Tsan Ho, Hong Kong (CN)
(74) Representative: Ullrich & Naumann PartG mbB

(57) **Abstract**

The present invention is related to an endoscopic ultrasonic cleaning adapter, adaptable to position in a port of an endoscope for assisting a cleaning procedure for said endoscope, comprising: a main body, comprising an internal cavity; a shaft, profiled into a tubular structure having at least five recesses and at least two openings thereon, wherein each of these recesses being fitted with a sealing member, and said at least two openings defining an internal channel inside said tubular structure allowing fluid passing therethrough, when said shaft being positioned in said port: said shaft reciprocally movable, in an accommodation cavity defined by said internal cavity of said main body and an internal cavity of said port of said endoscope, between a first position and a second position with respect to said port; said accommodation cavity including a bottom cavity defined by a region adjacent a bottom of said shaft and a region adjacent a bottom of said port; when said shaft at said first position, air flow in said cleaning procedure can pass through said internal channel but water flow in said cleaning procedure being impeded by said adapter; and when said shaft at said second position, water flow in said cleaning procedure can pass through said internal channel but air flow in said cleaning procedure being impeded by said adapter.

## Description

### FIELD OF THE INVENTION

The present invention is related to an endoscopic ultrasound (EUS) cleaning adapter, a specialized device designed for the critical reprocessing of EUS endoscopes.

### BACKGROUND OF THE INVENTION

The current EUS cleaning adapter's complex design is its primary weakness. Its intricate internal channels are prone to clogging and are extremely difficult to clean perfectly, creating a risk of bacterial contamination and infection between patients. This complexity also makes the device less reliable and more expensive.

The public needs a simpler design because it directly translates to enhanced patient safety. A design with fewer, more accessible pathways is easier to sterilize thoroughly, drastically reducing infection risk. This improved reliability ensures the device functions correctly during critical procedures and lowers costs for healthcare providers, making care both safer and more affordable. Simplification, in this case, is not a compromise but a significant advancement in medical safety and efficiency.

The present invention at least seeks to address issues of these problems, or at least to provide an alternative to the public.

### SUMMARY OF THE INVENTION

The first aspect of the present invention is related to an endoscopic ultrasonic cleaning adapter, adaptable to position in a port of an endoscope for assisting a cleaning procedure for said endoscope, comprising: a main body, comprising an internal cavity; a shaft, profiled into a tubular structure having at least five recesses and at least two openings thereon, wherein each of these recesses being fitted with a sealing member, and said at least two openings defining an internal channel inside said tubular structure allowing fluid passing therethrough; and when said shaft being positioned in said port: said shaft reciprocally movable, in an accommodation cavity defined by said internal cavity of said main body and an internal cavity of said port of said endoscope, between a first position and a second position with respect to said port; said accommodation cavity including a bottom cavity defined by a region adjacent a bottom of said shaft and a region adjacent a bottom of said port; when said shaft at said first position, air flow in said cleaning procedure can pass through said internal channel but water flow in said cleaning procedure being impeded by said adapter; and when said shaft at said second position, water flow in said cleaning procedure can pass through said internal channel but air flow in said cleaning procedure being impeded by said adapter.

In some embodiments, said adapter being configured to enable: when said shaft being at said first position, air flow, flowing into said port via a first conduit of said port, leaving said port via a third conduit and a fourth conduit of said port through said internal channel of said shaft; and water flow being impeded by said adapter to pass through said port; and when said shaft being at said second position, air flow being impeded by said adapter to pass through said port; and water flow, flowing into said port via a second conduit of said port, leaving said port via said third conduit and said fourth conduit.

In some embodiments, wherein when said shaft being positioned in said port, and when said shaft being at said first position, a first portion of air flow flowing into said port, via said first conduit, and leaving said port via said third conduit through said internal channel of said shaft; and/or a second portion of air flow flowing into said port, via said first conduit, and leaving said port via said fourth conduit through said internal channel of said shaft; and/or when said shaft being at said second position, a first portion of water flow flowing into said port, via said second conduit, and leaving said port via said third conduit through said internal channel of said shaft; and/or a second portion of water flow, flowing into said port, via said second conduit, and leaving said port via said fourth conduit through said bottom cavity.

In some embodiments, wherein said at least five recesses comprising a first recess, a second recess, a third recess, a fourth recess, and a fifth recess sequentially arranged from the top to said bottom of said shaft on the length side thereof.

In some embodiments, wherein when said shaft being positioned in said port, the sealing member for said first recess is always pressed or biased.

In some embodiments, wherein when said shaft being positioned in said port, the sealing member for said first recess is always pressed or biased by the inner side of the surrounding wall of said main body.

In some embodiments, wherein when said shaft being positioned in said port, the sealing member for said second recess is always pressed or biased.

In some embodiments, wherein when said shaft being positioned in said port, and when said shaft being at said first position, the sealing member for said second recess not impeding the air flow; and when said shaft being at said second position, the sealing member for said second recess impeding the air flow.

In some embodiments, wherein when said shaft being positioned in said port, and when said shaft being at said first position, the sealing member for said second recess being unbiased thus allowing the air flow, and when said shaft being at said second position, the sealing member for said second recess being biased to impede the air flow; or when said shaft being at said first position, the sealing member for said second recess being biased but still allowing the air flow, and when said shaft being at said second position, the sealing member for said second recess being biased to impede the air flow.

In some embodiments, wherein when said shaft being positioned in said port, and when said shaft being at said first position, the sealing member for said second recess being unbiased thus allowing the air flow, and when said shaft being at said second position, the sealing member for said second recess being biased by the inner side of the surrounding wall of said port, to impede the air flow; or when said shaft being at said first position, the sealing member for said second recess being biased by the inner side of the surrounding wall of said main body but still allowing the air flow, and when said shaft being at said second position, the sealing member for said second recess being biased by the inner side of the surrounding wall of said port, to impede the air flow.

In some embodiments, wherein one of said at least two openings of said shaft being provided on the length side of said tubular structure of said shaft, while another one of said at least two openings of said shaft being provided on said bottom of said tubular structure.

In some embodiments, wherein one of said at least two openings of said shaft being provided on the length side of said tubular structure of said shaft at a position higher than the third recess on said tubular structure, while another one of said at least two openings of said shaft being provided on said bottom of said tubular structure.

In some embodiments, wherein said internal channel of said shaft is T-shaped, such that a first opening and a second opening of said at least two openings being provided on opposite sides of the length side of said tubular structure of said shaft, and a third opening of said at least two openings being provided on said bottom of said tubular structure. The first and second and third openings are in fluidic communication.

In some embodiments, wherein said main body further comprising a limiting structure; and said shaft further comprising a limiting structure, such that when said shaft being positioned in said port, said limiting structure of said main body and said limiting structure of shaft holding said shaft at least partially received in said accommodation cavity.

In some embodiments, wherein the sealing member for said second recess of said shaft helping hold said shaft at least partially received in said accommodation cavity when said shaft being positioned in said port.

In some embodiments, wherein said shaft being made of plastic.

In some embodiments, wherein said shaft being manufactured as a single piece in plastic.

The second aspect of the present invention is related to a method to provide an endoscopic ultrasonic cleaning adapter of the first aspect of the present invention.

### DESCRIPTION OF THE DRAWINGS

Some embodiments of the present invention will now be explained, with reference to the accompanied drawings, in which:-
- Figure 1: is a perspective view of a first embodiment of a EUS cleaning adapter;
- Figure 2A: is a first sectional view of the first embodiment of the EUS cleaning adapter;
- Figure 2B: is a second sectional view of the first embodiment of the EUS cleaning adapter;
- Figure 3A: is a sectional view of the first embodiment of the EUS cleaning adapter mounted into a port of an endoscope, in which a cap of the EUS cleaning adapter is unpressed;
- Figure 3B: is a sectional view of the first embodiment of the EUS cleaning adapter mounted into a port of an endoscope, in which a cap of the EUS cleaning adapter is pressed;
- Figure 4A: is a sectional view of a second embodiment of the EUS cleaning adapter mounted into a port of an endoscope, in which a cap of the EUS cleaning adapter is unpressed; and
- Figure 4B: is a sectional view of the second embodiment of the EUS cleaning adapter mounted into a port of an endoscope, in which a cap of the EUS cleaning adapter is pressed.

### DETAILED DESCRIPTION OF PREFERRED EMBODIMENTS OF THE INVENTION

The present invention is now presented by way of examples with reference to the figures in the following paragraphs. Objects, features, and aspects of the present disclosure are disclosed in or are apparent from the following description. It should be understood by one of ordinary skilled in the art that the following description is a description of exemplary embodiments only, and is not intended as limiting the broader aspects of the present disclosure, which broader aspects are embodied in the exemplary constructions.

It should be noted that, unless otherwise defined, the technical terms or scientific terms used in the embodiments of the present invention shall have the usual meanings understood by person with ordinary skills in the art to which the present invention belongs. "First", "second" and similar expression used in the embodiments of the present invention do not indicate any order, quantity or importance, but are only used to distinguish different components. "Front", "rear", "left", "right", "upper", and "lower" and other terms indicating orientation or similar terms are only described for the exemplary relative positional relationship shown in the drawings to facilitate the understanding. It does not limit the disclosed components in the present invention can only follow this specific relative positional relationship.

For brevity's sake, the operation of cleaning an endoscope, the connection means of the endoscope to a water source, air source, and suction source are not described and explained in this specification, as this information constitute the state of art.

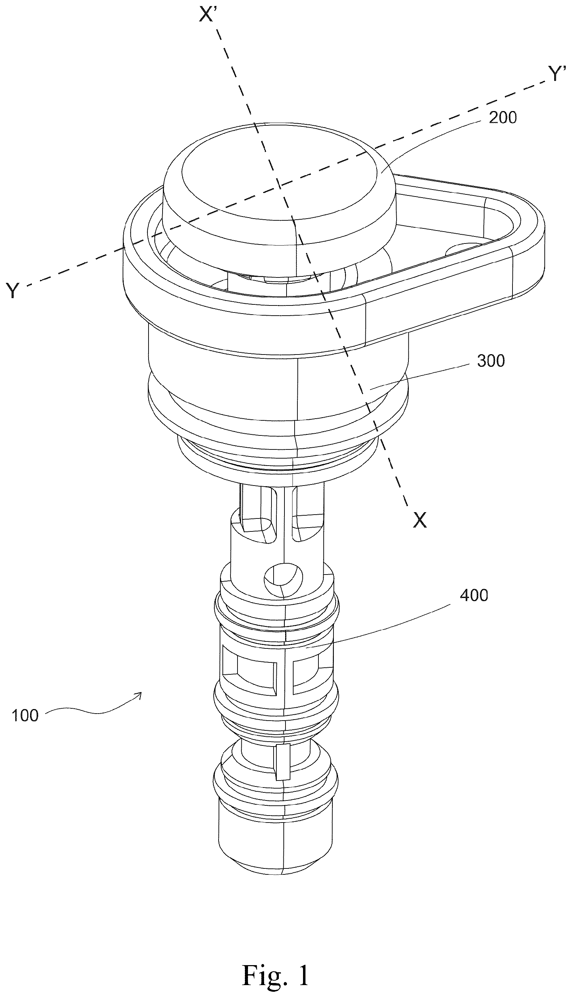

Figure 1 is a perspective view of a first embodiment of a EUS cleaning adapter 100, which comprises a cap 200, a main body 300, and a shaft 400.

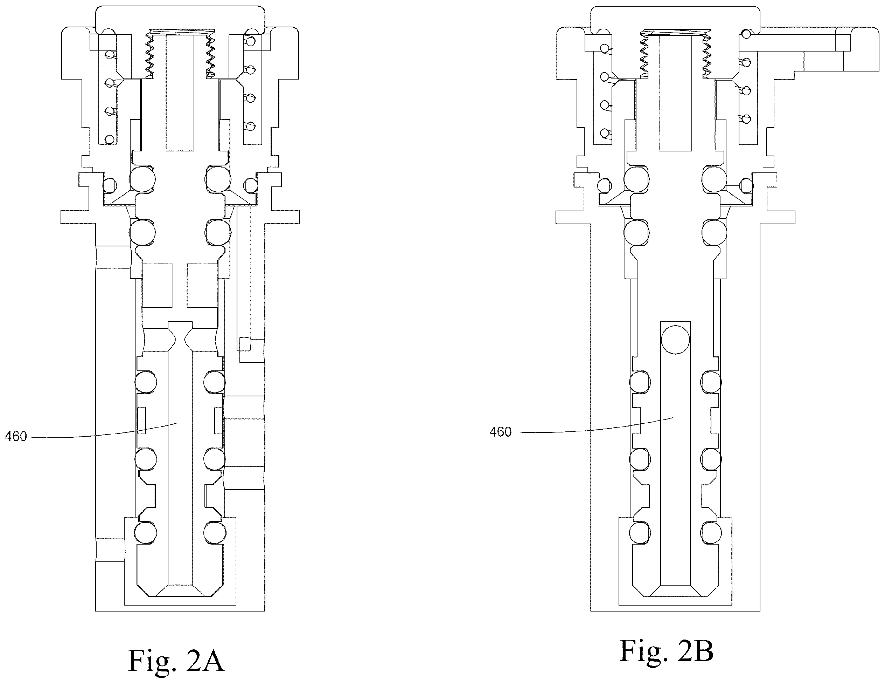

Figure 2A is a first sectional view, along dotted line XX' in figure 1, of the first embodiment of the EUS cleaning adapter.

Figure 2B is a second sectional view, along dotted line YY' in figure 1, of the first embodiment of the EUS cleaning adapter.

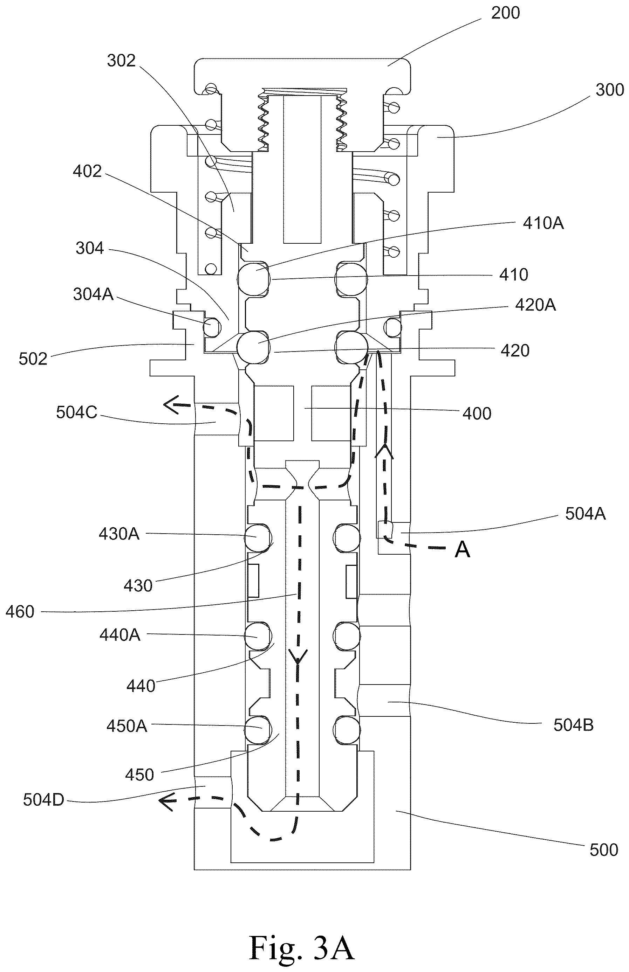

Figures 3A-3B are sectional views of the first embodiment of the EUS cleaning adapter 100 that is removably mounted into a port 500 which belongs to part an endoscope.

A resilient member is positioned between the cap 200 and the main body 300, in particular between the cap 200 and the main body's limiting structure 302. For brevity's sake, the resilient member is not shown in figures 3A-3B.

The cap 200 is mounted onto the upper portion the shaft 400 by means of a threaded screw, provided here as an illustrative example. For example, the cap 200 can also be mounted onto the upper portion the shaft 400 by means of snap fit and/or tight fit.

The main body 300 is designed to provide an inwardly protruding limiting structure 302 having a right-angled shape and extending from the surrounding wall of the main body 300, which defines an internal central cavity within the main body 300 that allows the shaft 400 to pass through. The main body 300 is further provided with an engagement structure 304. It shall be important to note that the shape of the limiting structure 302 and the engagement structure 304 disclosed in figures are examples only; those skilled in the art could configure it according to actual needs.

The shaft 400 has an elongate tubular structure and comprises an upper portion, a lower portion, and a limiting structure 402 outwardly protruding from the peripheral of the shaft 400. Preferably, the limiting structure 402 is configured as a right-angled profile that is complementary to the shape of the limiting structure 302 of the main body 300. It shall be important to note that the shape of the limiting structure 402 disclosed in figures is an example only; those skilled in the art could configure it according to actual needs. As long as the interaction of the limiting structure 302 and the limiting structure 402 limits the movement of the shaft 400, it belongs to the gist of the limiting structure.

The interaction between the limiting structure 302 of the main body 300 and the limiting structure 402 of the shaft 400 defines the upper portion and the lower portion of the shaft 400, in which the portion of the shaft 400 above said interaction is the upper portion of the shaft 400 while the portion of the shaft 400 below said interaction is the lower portion of the shaft 400. In other words, the portion above the limiting structure 402 is considered as the upper portion of the shaft 400, and the portion below the limiting structure 402 together with the limiting structure 402 is considered as the lower portion of the shaft 400.

The port 500 has a receptacle-like structure with an open-top internal cavity defined by the surrounding wall and bottom of the port 500. The port 500 is configured to receive the EUS cleaning adapter 100 and is provided with an engagement structure 502.

The main body 300 is in direct contact with the port 500, as shown in figures. The main body 300 and port 500 are assembled by engaging their respective engagement structures 304 and 502, for example, via a snug fit. Between these engagement structures 304 and 502 is provided with a sealing member 304A for preventing leakage and for enhancing the engagement between these engagement structures 304 and 502.

The shaft 400 is at least partially housed inside an accommodation cavity defined by the internal cavity of the main body 300 and the internal cavity of the port 500. The depth of the internal cavity of the port 500 is deeper than the length of the lower portion of the shaft 400, such that the lower portion of the shaft 400 is movable in the internal cavity of the port 500 between the limiting structure 302 of the main body 300 and the bottom of the port 500. To be more specific, with the aid of the resilient member, disposed between the cap 200 and the main body 300, the lower portion of the shaft 400 is reciprocally movable between the limiting structure 302 of the main body 300 and the bottom of the port 500. Advantageously, regardless whether the cap 200 is pressed or not pressed, the bottom of the shaft 400 does not engage with the bottom of the port 500.

The position of the cap 200 with respect to the port 500 controls a pressing action. When a user presses the cap 200, it actuates the lower portion of the shaft 400 to move from a first position where the limiting structure 302 of the main body 300 interacts with the limiting structure 402 of the shaft 400 and the lower portion of the shaft 400 does not engage with the bottom of the port 500 to a second position where the limiting structure 302 of the main body 300 does not interact with the limiting structure 402 of the shaft 400. By default, when the cap 200 is unpressed, the cap 200 is at its first position; when the cap 200 is pressed, the cap 200 is at its second position. The position of the cap 200 with respect to the port 500 also defines the configuration of the EUS cleaning adapter 100, in which the first position of the cap 200 defines the first configuration of the EUS cleaning adapter 100 and the second position of the cap 200 defines the second configuration of the EUS cleaning adapter 100.

The shaft 400 features a sophisticated sealing system comprising five precisely machined recesses-designated as a first recess 410, a second recess 420, a third recess 430, a fourth recess 440, and a fifth recess 450-arranged sequentially downwards along the vertical axis ofthe shaft, in which the first recess 410 is the top recess among all recesses. Preferably, the first recess 410 is always in the cavity defined by the main body 300 during the operation of cleaning an endoscope. More preferably, the third recess 430, the fourth recess 440, and the fifth recess 450 are always in the cavity defined by the port 500 during the operation of cleaning an endoscope. It shall be important to note that the number of recesses provided to the shaft 400 is at least five. Each recess is annularly, preferably continuous, around the tubular structure of the shaft 400 and hosts a sealing member: a first sealing member 410A, a second sealing member 420A, a third sealing member 430A, a fourth sealing member 440A, and a fifth sealing member 450A, respectively. The first recess 410 and second recess 420 are advantageously designed with greater width compared to the third recess 430, fourth recess 440, and fifth recess 450, allowing them to accommodate larger or more robust sealing elements. However, larger first recess 410 and second recess 420 are not necessary to practice the invention.

Integral to the shaft's function is a T-shaped internal channel 460, which serves as a multifunctional fluid conduit. It shall be important to note that although the internal channel 460 is T-shaped in figures, it is for illustration only. The essence of the internal channel of the present invention is to provide a tunnel with one first fluid outlet and one second fluid inlet. As shown in figures, the internal channel 460 comprises a horizontal profile and a vertical profile, which are in fluidic communication. The horizontal profile of the internal channel 460 is positioned higher than the third recess 430 but lower than the second recess 420. From this horizontal profile of the internal channel 460, the vertical profile of the internal channel 460 extends downward, traversing the zones associated with the third recess 430, fourth recess 440, and fifth recess 450, and terminating at the bottom (or distal end) of the shaft 400. This configuration of the internal channel enables the internal channel 460 to function as a controlled passage for fluids, likely facilitating the directed flow of cleaning fluids during cleaning. It shall be important to note that the internal channel 460 is not necessary to be T-shaped. As long as an internal channel that has at least two openings, in which one of the two openings is provided on the length side of the tubular structure of the shaft at a position higher than the third recess 430/third sealing member 430A but lower than the second recess 420/second sealing member 420A while another one of the two openings is provided on the bottom of the tubular structure of the shaft, it also falls into the gist of the internal channel. In some embodiments, the shape of the internal channel can be Y-shaped, inverted A-shaped, or any shape. In some embodiments, the internal channel has three, four, five or even more openings according to actual needs.

As shown in figures, the sealing member 304A, the first sealing member 410A, the second sealing member 420A, the third sealing member 430A, the fourth sealing member 440A, and the fifth sealing member 450A each is configured as a ring structure having a central bore to sleeve around the tubular structure of the shaft 400 in their respective recesses. It is important to note that these sealing members are sized and dimensioned fit to act against a side facing opposite to these sealing members, such as the inner side of the surrounding wall of the port, avoiding contact between the surface of the tubular structure of the shaft and the surface of the inner side of the surrounding wall of the port thus preventing wearing off the surface of the inner side of the surrounding wall of the port. As long as the sealing member is sized and dimensioned to act against the inner side of the surrounding wall of the port, avoiding contact between the surface of the tubular structure of the shaft and the surface of the inner side of the surrounding wall of the port thus preventing wearing off the surface of the inner side of the surrounding wall of the port, such alternative of the sealing member also falls within the gist of the invention.

The surrounding wall of the port 500 is profiled to provide a first conduit 504A for receiving air flow from an air source for the endoscope, a second conduit 504B for receiving water flow from a water source for the endoscope, a third conduit 504C, and a fourth conduit 504D. Both third conduit 504C and fourth conduit 504D are outlet ports for air and/or water. It shall be important to understand that the use of air and water is for explanation the operation of the EUS cleaning adapter and shall not be construed as limitation to the use of the EUS cleaning adapter. In some embodiments, the first conduit 504A can be configured for receiving water flow from a water source for the endoscope, a second conduit 504B can be configured for receiving air flow from an air source for the endoscope. It shall also be important to understand that "conduit" in context means a channel or tunnel or through-opening allowing fluid, i.e., liquid and/or air, passing through. "Fluid" in this specification may refer to liquid or air. For example, the first conduit 504A is for allowing fluid to pass through the surrounding wall of the port 500. It is important to note that the conduit shall not be constrained as any shape. Advantageously, the internal cavity of the port 500 is enlarged in the region near the fourth conduit 504D, where the spacing between the inner side of the port's surrounding wall and the outer side of the shaft's surrounding wall is greater than in other regions of the port's internal cavity.

When the EUS cleaning adapter 100 is mounted to the port 500, the operation of the EUS cleaning adapter 100, i.e., pressing or not pressing the cap 200 of the EUS cleaning adapter 100, operably allows the first conduit 504A and the second conduit 504B to allow air/water to pass through the EUS cleaning adapter 100 to the third conduit 504C and the fourth conduit 504D. If it allows, air flowing from the first conduit 504A to the third conduit 504C and/or to the fourth conduit 504D through the internal channel 460 is enabled; and water flowing from the second conduit 504B to the third conduit 504C through the internal channel 460, and/or to the fourth conduit 504D through the channel defined by the bottom of the shaft and bottom of the port 500 is enabled.

When the EUS cleaning adapter 100 is mounted to the port 500, the outer side of the surrounding wall of the shaft 400 and the inner side of the surrounding wall of the port 500 leaves a narrow channel running their entire lengths, which functions as a dedicated fluid passage. The sealing members 410A, 420A, 430A, 440A, and 450A, made of resilient materials and mounted to their respective recesses of the tubular structure of the shaft 400, acts against the inner side of the surrounding wall of the port 500 thus blocking and/or impeding the narrow channel, i.e., the dedicated fluid passage. In this specification, "narrow channel between the outer side of the surrounding wall of the shaft 400 and the inner side of the surrounding wall of the port 500" and "dedicated fluid passage" are interchangeable. It shall be important to understand that "the sealing member acts the inner side of the surrounding wall of the port" in context can also be understood as "the inner side of the surrounding wall of the port biases or presses or compresses the sealing member". When the sealing member is biased, it provides a sealing effect.

Figure 3A depicts the EUS cleaning adapter 100 is mounted to the port 500, in which the cap 200 is not pressed, the first sealing member 410A acts against the inner side of the surrounding wall of the port 500, and the second sealing member 420A does not act against the inner side of the surrounding wall of the port 500. Therefore, during the cleaning step of the endoscope, when the cap 200 is not pressed, air (denoted with letter A in figure 3A) flushes into the first conduit 504A then leaves therefrom, then enters the narrow channel between the exit of the first conduit 504A and the second sealing member 420A, then enters the narrow channel between the inner side of the surrounding wall of the port 500 and the outer side of the surrounding wall of the shaft 400, then enters the internal channel 460. Inside the internal channel 460, the flushed-in air bifurcates into two paths: the first path - the flushed air flows through the horizontal profile of the internal channel 460, then enters the narrow channel between the inner side of the surrounding wall of the port 500 and the outer side of the surrounding wall of the shaft 400, then leaves the port 500 via the third conduit 504C; and the second path - the flushed air flows to the junction between the horizontal profile and the vertical profile of the internal channel 460 then flows to the vertical profile of the internal channel 460, then enters the cavity between bottom of the shaft 400 and the bottom of the port 500, then enters the narrow channel between the inner side of the surrounding wall of the port 500 and the outer side of the surrounding wall of the shaft 400, then leaves the port 500 via the fourth conduit 504D.

Since the first sealing member 410A acts against the inner side of the surrounding wall of the main body 300 and the third sealing member 430A acts against the inner side of the surround wall of the main body 300, it can prevent air from leaving the main part 300 and air can only be directed to flow through the EUS cleaning adapter 100 with path as described above.

In figure 3A, during the cleaning step of the endoscope, water flushes into the second conduit 504B. Since the fourth sealing member 440A and the fifth sealing member 450A act against the inner side of the surrounding wall of the port 500, blocking and/or impeding the narrow channel between the outer side of the surrounding wall of the shaft 400 and the inner side of the surrounding wall of the port 500, therefore water fails to pass through the EUS cleaning adapter 100 and stops at the second conduit 504B.

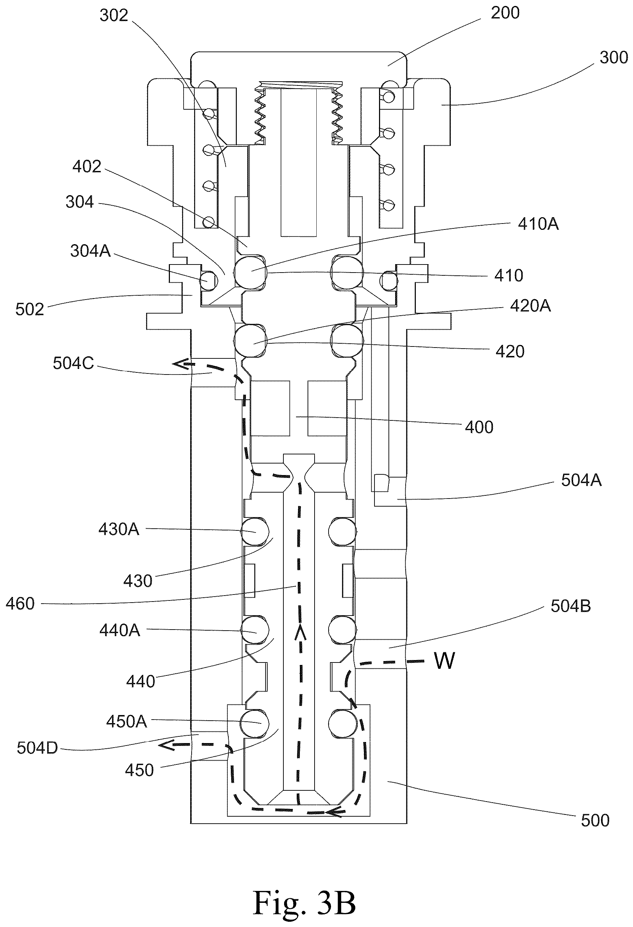

Figure 3B depicts that the EUS cleaning adapter 100 is mounted to the port 500, in which the cap 200 is pressed and the first and second sealing members 410A and 420A act against the inner side of the surrounding wall of the main body 300 and the inner side of the surrounding wall of the port 500, respectively. Therefore, during the cleaning step of the endoscope when the cap 200 is pressed, air flushes into the first conduit 504A. Since the second sealing member 420A acts against the inner side of the surrounding wall of the port 500, blocking and/or impeding the narrow channel between the outer side of the surrounding wall of the shaft 400 and the inner side of the surrounding wall of the port 500, therefore air fails to pass through the EUS cleaning adapter and stops at the first conduit 504A.

In figure 3B, during the cleaning step of the endoscope, water (denoted with letter W in figure 3B) flushes into the second conduit 504B. Due to the enlarged internal cavity of the port 500 at a region close to the fourth conduit 504D, water flows through the narrow channel to fourth conduit 504D through the cavity between the bottom of the shaft 400 and the bottom of the port 500 and the cavity between the outer side of the surrounding wall of the shaft 400 and the inner side of the surrounding wall of the port 500. At there, the water bifurcates into two paths: the first path - the water flows into the vertical profile of the internal channel 460, then enters the junction between the horizontal profile and the vertical profile of the internal channel 460 then flows to the cavity between the outer side of the surrounding wall of the shaft 400 and the inner side of the surrounding wall of the port 500, then leaves the port 500 via the third conduit 504C; and the second path - the water flows to the cavity between the bottom of the shaft 400 and the bottom of the port 500, then enters the narrow channel between the inner side of the surrounding wall of the port 500 and the outer side of the surrounding wall of the shaft 400, then leaves the port 500 via the fourth conduit 504D. Since the fourth sealing member 440A acts against the inner side of the surrounding wall of the port 500, blocking and/or impeding the narrow channel between the fourth sealing member 440A and the inner side of the surrounding wall of the port 500.

With description above, it shall be understood that the first sealing member 410A always acts against the inner side of the surrounding wall of the main body 300 regardless whether the cap 200 is pressed or not. While for the second sealing member 420A, it acts against the inner side of the surrounding wall of the port 500 when the cap 200 is pressed, while it does not act against the inner side of the surrounding wall of the port 500 when the cap 200 is not pressed.

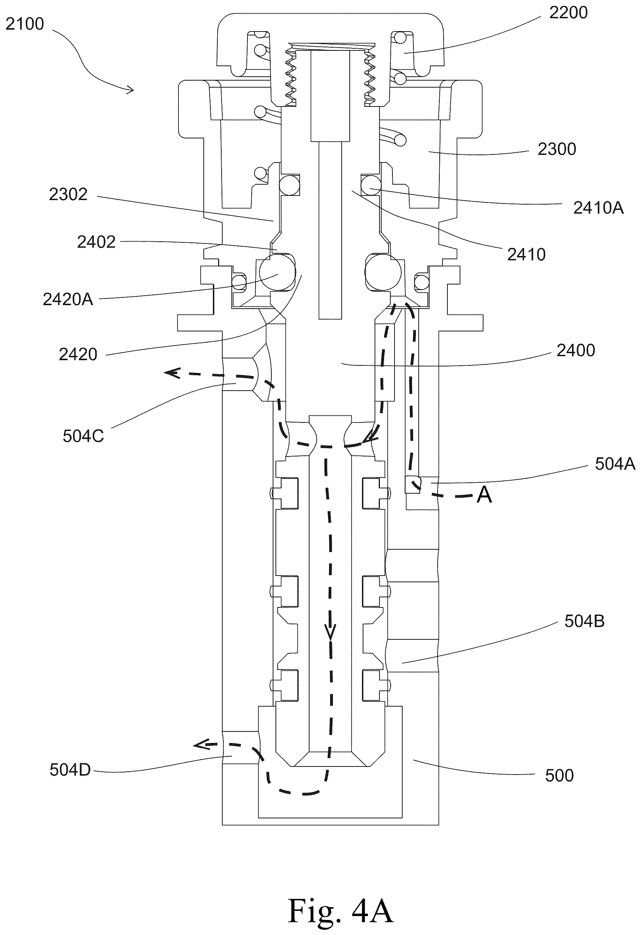

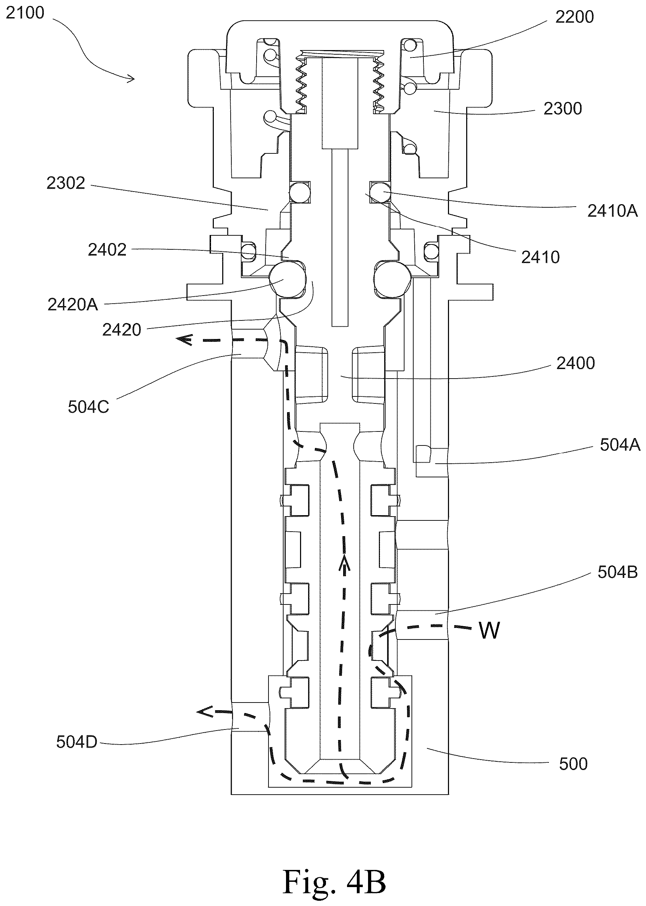

Figures 4A-4B depict a second embodiment of the EUS cleaning adapter 2100 according to the present disclosure. The EUS cleaning adapter 2100 is generally the same as the EUS cleaning adapter 100 in particular the flow of air (dotted line denoted by letter A in figure 4A) and/or water (dotted line denoted by letter W in figure 4B) through the port or through the port and the EUS cleaning adapter according to the unpressed and pressed condition of the cap of the EUS cleaning adapter. For sake of brevity and clarity, only major differences are illustrated.

Figures 4A-4B are sectional views of the EUS cleaning adapter 2100 that is mounted into a port 500 which belongs to part an endoscope, in which the EUS cleaning adapter 2100 comprises a cap 2200, a main body 2300 having a guiding structure 2302, and a shaft 2400 having a guiding structure 2402 complementary to the guiding structure 2302 for guiding the movement of the shaft 2400. Although the figures show that these guiding structures 2302 and 2402 have slanted profiles, it shall not be construed as limitation to the gist of the guiding structure.

The first main difference between the EUS cleaning adapter 100 and the EUS cleaning adapter 2100 is that, as clearly shown in figures, the limiting structures to limit the movement of the shaft inside the accommodation cavity defined by the internal cavity of the main body 300 and the internal cavity of the port 500 are the second sealing member 2420A and the main body 2300, preferably the surrounding wall of the main body 2300, more preferably the inner side of the surrounding wall of the main body, whereas in the EUS cleaning adapter 100, its limiting structures are the limiting structure 302 of the main body 300 and the limiting structure 402 of the shaft 400, as described above.

The second main difference lies in that a first recess 2410 and a first sealing member 2410A are both positioned in an upper portion of the shaft 2400 according to the aforementioned definition for figures 3A and 3B. In contrast, in the EUS cleaning adapter 100, the first recess 410 and the first sealing member 410A are both positioned in the lower portion of the shaft 400 according to the same definition.

The third main difference lies in that in the unpressed condition of the cap 2200 in the EUS cleaning adapter 2100, the second sealing member 2420A acts against the inner side of the surrounding wall of the main body 2300 or in other words, the second sealing member 2420A is pressed or compressed or biased by the inner side of the surrounding wall of the main body 2300. While in the pressed condition of the cap 2200, the second sealing member 2420A acts against the port 500 preferably the surrounding wall of the port 500 or more preferably the inner side of the surrounding wall of the port 500, or in other words, the second sealing member 2420A is pressed or compressed or biased by the inner side of the surrounding wall of the port 500. In contrast, in the unpressed condition of the cap 200 in the EUS cleaning adapter 100, the second sealing member 420A is free from press or compress or bias, and it is pressed or compressed or biased by the inner side of the surrounding wall of the port 500 when the cap 200 is in a pressed condition.

It is worth noting the first sealing member 410A in the EUS cleaning adapter 100 and the first sealing member 2410A in the EUS cleaning adapter 2100 are always pressed or biased, by the inner side of the surrounding wall of their respective main bodies, regardless of the press condition of their respective caps 200 and 2200.

Depending on the application and design, any one of the sealing members-sealing member 304A, the first sealing member 410A, the second sealing member 420A, the third sealing member 430A, the fourth sealing member 440A, or the fifth sealing member 450A, as shown in Figures 3A and 3B-can be alternatively configured to comprise a central bore and an annular protrusion projecting outwardly from the surrounding wall of the central bore. The annular protrusion is sized and dimensioned to act against a side facing opposite to the protrusion, such as the inner side of the surrounding wall of the port. Alternative embodiments of these sealing members are illustrated, for example, as the third, fourth, and fifth sealing members in Figures 4A and 4B. It shall be important to note that the annular protrusion is sufficiently long to act against the inner side of the surrounding wall of the port, avoiding contact between the surface of the tubular structure of the shaft and the surface of the inner side of the surrounding wall of the port thus preventing wearing off the surface of the inner side of the surrounding wall of the port. As long as the sealing member is sized and dimensioned to act against the inner side of the surrounding wall of the port, avoiding contact between the surface of the tubular structure of the shaft and the surface of the inner side of the surrounding wall of the port thus preventing wearing off the surface of the inner side of the surrounding wall of the port, these alternative of the sealing member also falls within the gist of the invention.

Due to the novel structural design of the EUS cleaning adapter described in the present invention, the shaft can be manufactured as a single, integrated piece with a simplified configuration. This streamlined design enables the use of plastic as the primary material for fabrication, which offers several advantages over conventional designs. In contrast, the shafts used in prior art EUS cleaning adapters typically consist of multiple assembled components and are often fabricated from metal. Such multi-piece metal constructions not only increase manufacturing complexity and associated production costs, but also require additional steps for assembly and post-use cleaning. By utilizing plastic materials, the shaft of the EUS cleaning adapter of the present invention can be designed for single-use, thereby significantly improving hygiene and reducing the risk of cross-contamination. In some embodiments, the shaft, the cap, the main body all can be fabricated in plastic. Furthermore, the disposable nature of the device eliminates the need for time-consuming cleaning and sterilization procedures associated with reusable metal shafts. This advancement not only simplifies the manufacturing process but also enhances operational efficiency and safety in clinical settings.

The above-described shall not be interpreted to be restricted by the examples or figures only. It is to be expressly understood, however, that such modifications and adaptations are within the scope of invention in this aspect. For instance, features illustrated or described as part of one embodiment can be used on another embodiment to yield a still further embodiment. Thus, it is intended that the present disclosure cover such modifications and variations and their equivalents.

## Claims

1. An endoscopic ultrasonic cleaning adapter, adaptable to position in a port of an endoscope for assisting a cleaning procedure for said endoscope, comprising:
a main body, comprising an internal cavity;
a shaft, profiled into a tubular structure having at least five recesses and at least two openings thereon, wherein
each of these recesses being fitted with a sealing member, and
said at least two openings defining an internal channel inside said tubular structure allowing fluid passing therethrough; and
**characterized in that**, when said shaft being positioned in said port:
said shaft reciprocally movable, in an accommodation cavity defined by said internal cavity of said main body and an internal cavity of said port of said endoscope, between a first position and a second position with respect to said port;
said accommodation cavity including a bottom cavity defined by a region adjacent a bottom of said shaft and a region adjacent a bottom of said port;
when said shaft at said first position, air flow in said cleaning procedure can pass through said internal channel but water flow in said cleaning procedure being impeded by said adapter; and
when said shaft at said second position, water flow in said cleaning procedure can pass through said internal channel but air flow in said cleaning procedure being impeded by said adapter.

2. The adapter according to claim 1, said adapter being configured to enable:
when said shaft being at said first position, air flow, flowing into said port via a first conduit of said port, leaving said port via a third conduit and a fourth conduit of said port through said internal channel of said shaft; and water flow being impeded by said adapter to pass through said port; and
when said shaft being at said second position, air flow being impeded by said adapter to pass through said port; and water flow, flowing into said port via a second conduit of said port, leaving said port via said third conduit and said fourth conduit.

3. The adapter according to claim 2, wherein when said shaft being positioned in said port, and
when said shaft being at said first position,
a first portion of air flow flowing into said port, via said first conduit, and leaving said port via said third conduit through said internal channel of said shaft; and/or
a second portion of air flow flowing into said port, via said first conduit, and leaving said port via said fourth conduit through said internal channel of said shaft; and/or
when said shaft being at said second position,
a first portion of water flow flowing into said port, via said second conduit, and leaving said port via said third conduit through said internal channel of said shaft; and/or
a second portion of water flow, flowing into said port, via said second conduit, and leaving said port via said fourth conduit through said bottom cavity.

4. The adapter according to claim 1, wherein said at least five recesses comprising a first recess, a second recess, a third recess, a fourth recess, and a fifth recess sequentially arranged from the top to said bottom of said shaft on the length side thereof.

5. The adapter according to claim 4, wherein when said shaft being positioned in said port, the sealing member for said first recess is always pressed or biased, preferably by the inner side of the surrounding wall of said main body.

6. The adapter according to claim 5, wherein when said shaft being positioned in said port, the sealing member for said second recess is always pressed or biased.

7. The adapter according to claim 5, wherein when said shaft being positioned in said port, and
when said shaft being at said first position, the sealing member for said second recess not impeding the air flow; and
when said shaft being at said second position, the sealing member for said second recess impeding the air flow.

8. The adapter according to claim 7, wherein when said shaft being positioned in said port, and
when said shaft being at said first position, the sealing member for said second recess being unbiased thus allowing the air flow, and when said shaft being at said second position, the sealing member for said second recess being biased to impede the air flow; or
when said shaft being at said first position, the sealing member for said second recess being biased but still allowing the air flow, and when said shaft being at said second position, the sealing member for said second recess being biased to impede the air flow.

9. The adapter according to claim 7, wherein when said shaft being positioned in said port, and
when said shaft being at said first position, the sealing member for said second recess being unbiased thus allowing the air flow, and when said shaft being at said second position, the sealing member for said second recess being biased by the inner side of the surrounding wall of said port, to impede the air flow; or
when said shaft being at said first position, the sealing member for said second recess being biased by the inner side of the surrounding wall of said main body but still allowing the air flow, and when said shaft being at said second position, the sealing member for said second recess being biased by the inner side of the surrounding wall of said port, to impede the air flow.

10. The adapter according to claim 5, wherein one of said at least two openings of said shaft being provided on the length side of said tubular structure of said shaft, while another one of said at least two openings of said shaft being provided on said bottom of said tubular structure.

11. The adapter according to claim 5, wherein one of said at least two openings of said shaft being provided on the length side of said tubular structure of said shaft at a position higher than the third recess on said tubular structure, while another one of said at least two openings of said shaft being provided on said bottom of said tubular structure.

12. The adapter according to 10, wherein said internal channel of said shaft is T-shaped, such that a first opening and a second opening of said at least two openings being provided on opposite sides of the length side of said tubular structure of said shaft, and a third opening of said at least two openings being provided on said bottom of said tubular structure.

13. The adapter according to any one of claims 1-12, wherein
said main body further comprising a limiting structure; and
said shaft further comprising a limiting structure,
such that when said shaft being positioned in said port, said limiting structure of said main body and said limiting structure of shaft holding said shaft at least partially received in said accommodation cavity.

14. The adapter according to any one of claims 5-13, wherein
the sealing member for said second recess of said shaft helping hold said shaft at least partially received in said accommodation cavity when said shaft being positioned in said port.

15. The adapter according to any one of claims 1-14, wherein said shaft being made of plastic, preferably as a single piece.
